# DEMANDE DE BREVET EUROPEEN

(11) **EP 2 840 519 A1**
(43) Date de publication de la demande: **25.02.2015**
(21) Numéro de dépôt: 14181528.2
(22) Date de dépôt: 20.08.2014
(51) Int. Cl.: G06F 19/00, A63B 69/38

(54) **Procédé de traitement de données représentatives des performances d'un joueur de tennis**

(30) Priorité: 21.08.2013 FR 1358106
(71) Demandeur: BABOLAT VS, 69007 Lyon (FR)
(72) Inventeur: Mace, Pierre, 69007 Lyon (FR); Gauthier, Fabien, 69007 Lyon (FR)
(74) Mandataire: Myon, Gérard Jean-Pierre

(57) **Abrégé**

Ce procédé de traitement de données représentatives des performances d'un joueur de tennis dans un dispositif d'affichage comprend des étapes consistant à a) calculer, à partir de données comprenant des grandeurs physiques mesurées au cours d'une période de jeu comprenant une ou plusieurs sessions de jeu et téléchargées dans un système de calcul du dispositif d'affichage, au moins trois paramètres (P1, P2, P3) représentatifs des performances du joueur lors de cette période de jeu, b) afficher, sous la forme d'un diagramme comprenant un axe pour chaque paramètre (P1, P2, P3), les axes formant une étoile à plusieurs branches, des points correspondant aux valeurs (V1, V2, V3) des paramètres sur les axes de manière à former un contour fermé (A) de forme globalement polygonale englobant ou passant par lesdits points, et c) augmenter (A2) et diminuer (A1) alternativement l'aire délimitée par le contour fermé (A) selon une fréquence de variation calculée en fonction du temps de jeu effectif du joueur mesuré au cours de la dernière session de jeu de la période de jeu.

## Description

L'invention concerne un procédé de traitement de données représentatives des performances d'un joueur de tennis sur un dispositif d'affichage.

Pour la pratique du tennis, il est utile d'avoir un moyen de visualisation de l'évolution des performances d'un joueur au cours du temps, notamment avec un dispositif d'affichage portatif, tel qu'un téléphone mobile, une tablette ou un ordinateur portable. Des capteurs implantés dans la raquette permettent de mesurer un certain nombre de paramètres, notamment le nombre de coups et les caractéristiques de ces coups, et de les transférer dans un dispositif d'affichage portable. Cependant, les performances d'un joueur peuvent être représentées de multiples façons et à l'aide de multiples indices, ce qui rend difficile une visualisation claire et synthétique.

L'objet de l'invention propose un nouveau procédé de traitement de données représentatives des performances d'un joueur de tennis permettant de visualiser, par des techniques de traitement et d'affichage particulières, un certain nombre de paramètres grâce auxquels le joueur ou son entourage peut avoir un aperçu global de ses performances.

A cet effet, l'invention concerne un procédé de traitement de données représentatives des performances d'un joueur de tennis par un dispositif d'affichage, caractérisé en ce qu'il comprend des étapes consistant à :
- a) calculer, à partir de données comprenant des grandeurs physiques mesurées au cours d'une période de jeu comprenant une ou plusieurs sessions de jeu et téléchargées dans un système de calcul du dispositif d'affichage, au moins trois paramètres représentatifs des performances du joueur lors de cette période de jeu,
- b) afficher, sous la forme d'un diagramme comprenant un axe pour chaque paramètre, les axes formant une étoile à plusieurs branches, des points correspondant aux valeurs des paramètres sur les axes de manière à former un contour fermé de forme globalement polygonale englobant ou passant par lesdits points,
- c) augmenter et diminuer alternativement l'aire délimitée par le contour fermé selon une fréquence de variation calculée en fonction du temps de jeu effectif du joueur mesuré au cours de la dernière session de jeu de la période de jeu.

Grâce à l'invention, un joueur de tennis peut visualiser rapidement, grâce à un coup d'oeil sur un écran de son dispositif d'affichage, un aperçu de ses performances basé sur au moins trois paramètres et en apprécier la pertinence grâce aux variations de l'aire délimitée par le contour fermé, qui reflètent la fréquence de jeu du joueur.

Selon des caractéristiques avantageuses mais non obligatoires de l'invention, un tel procédé peut incorporer une ou plusieurs des caractéristiques suivantes, prises dans toute combinaison techniquement admissible :
- Le procédé comprend des étapes supplémentaires consistant à :
   - d) déterminer, pour chaque coup réalisé lors de la période de jeu, le type de ce coup parmi une liste de types de coups prédéfinie comportant au moins deux types de coups,
   - e) comptabiliser, pour chaque type de coup de la liste de coups, le nombre total de coups réalisés ;
   - f) colorer une surface délimitée par le contour fermé dans une couleur représentative du niveau du joueur, ce niveau étant déterminé en fonction du nombre total de coups réalisés pour chaque type de coup de la liste de types de coups.
- La liste de types de coups comprend au moins quatre types de coup, parmi lesquels le coup droit, le revers, le service et le smash.
- Le procédé de traitement comprend une étape consistant à signaler l'atteinte par le joueur d'un nombre prédéfini de coups pour chaque type de coup par un changement de couleur de la surface délimitée par le contour fermé et l'adoption d'une teinte correspondant à un niveau supérieur par rapport au niveau dans lequel le joueur était avant d'atteindre les nombres de coups prédéfinis pour chaque type de coup.
- Les paramètres calculés à l'étape a) sont respectivement représentatifs de la puissance développée par le joueur au cours de la période de jeu, de l'endurance du joueur au cours de la période de jeu et des aptitudes techniques du joueur au cours de la période de jeu, et le contour fermé a une forme globalement triangulaire.
- La fréquence de variation de l'aire délimitée par le contour fermé est limitée à une valeur maximale qui est atteinte lorsque le temps de jeu effectif lors de la dernière session de jeu de la période de jeu atteint une valeur seuil.
- La fréquence de variation de l'aire délimitée par le contour fermé décroît lorsque le joueur ne télécharge pas de données pendant une période de temps prédéterminée.
- Lorsque la fréquence de variation devient nulle, les nombres totaux de coups pour chaque type de coup commencent à décroitre.
- Chaque fois que des données concernant une nouvelle session de jeu sont téléchargées par le joueur dans le dispositif d'affichage, les variations de l'aire du contour fermé sont prolongées pendant une période de temps calculée en fonction du temps de jeu effectif mesuré pendant cette dernière session de jeu.
- Le procédé comprend une étape consistant à calculer une valeur moyenne des au moins trois paramètres calculés à l'étape a) et une étape consistant à comparer, au moyen d'un réseau de données, les performances de différents joueurs de tennis sur la base de cette valeur moyenne.

L'invention sera mieux comprise et d'autres avantages de celle-ci apparaitront plus clairement à la lumière de la description qui va suivre d'un procédé conforme à son principe, faite à titre d'exemple non limitatif en référence aux dessins annexés dans lesquels :

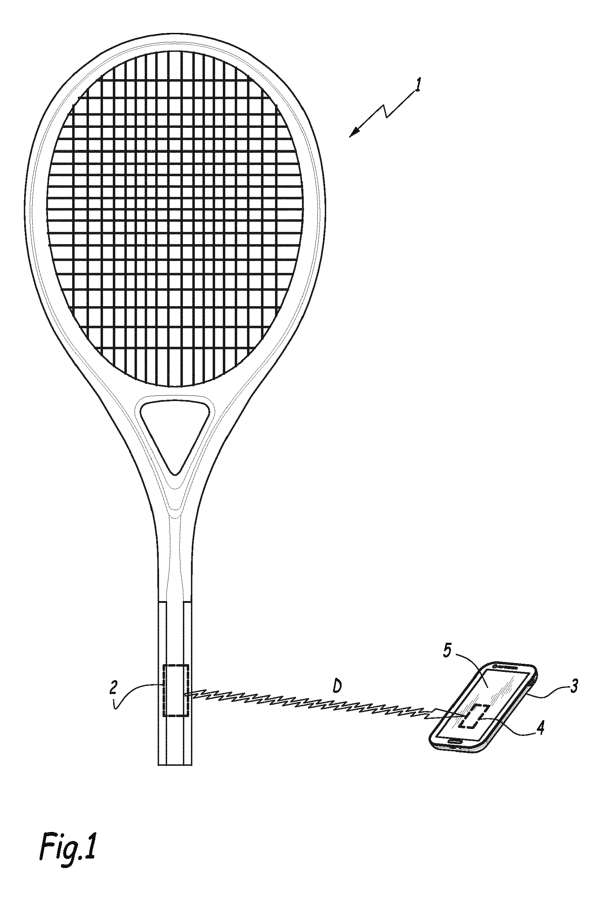

- la figure 1 est une vue schématique d'une raquette de tennis et d'un dispositif d'affichage permettant la mise en oeuvre du procédé conforme à l'invention ;
- la figure 2 est une représentation d'un affichage obtenu par un premier mode de réalisation du procédé conforme à l'invention ;
- la figure 3 est une représentation d'un affichage obtenu par un second mode de réalisation du procédé conforme à l'invention ;

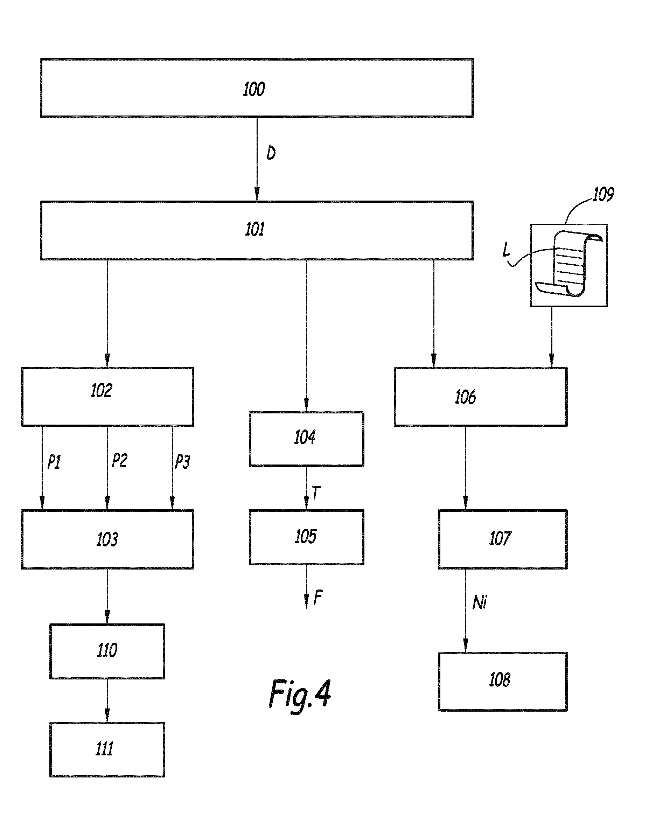

- la figure 4 est un schéma bloc du procédé conforme à l'invention.

Le procédé de l'invention est mis en oeuvre avec une raquette de tennis 1 équipée d'un système 2 de mesures de données de jeu D. Le système de mesure 2 comprend des capteurs non représentés aptes à mesurer, lors de la frappe d'une balle avec la raquette 1, des données D comprenant des grandeurs physiques telles que l'accélération linéaire et la vitesse angulaire de la raquette 1 selon trois directions perpendiculaires entre elles et fixes par rapport au référentiel terrestre. Par exemple, le système de mesure 2 peut comprendre un accéléromètre tri-axes et un gyromètre tri-axes.

Lors d'une période de jeu comprenant plusieurs sessions de jeu, les données de jeu D sont mesurées, dans une première étape 100 du procédé selon l'invention, par le système de mesure 2 lorsqu'un joueur réalise des séries de coups (coup droit lifté, service, revers slicé ...).

Lorsque le joueur termine une session de jeu, il connecte sa raquette 1 à un dispositif d'affichage 3, tel qu'un ordinateur, une tablette ou un téléphone mobile, de manière à télécharger, dans une étape 101, les données D recueillies à l'étape 100, dans le dispositif d'affichage qui, dans l'exemple représenté, est un téléphone mobile 3.

La connexion entre le dispositif d'affichage 3 et la raquette 1 peut être réalisée par l'intermédiaire d'une connexion filaire, par exemple de type USB, ou une connexion sans fil, par exemple de type Bluetooth.

Le dispositif d'affichage 3 comporte un système de calcul 4 intégré apte à calculer automatiquement, dans une étape 102, et à partir des données D téléchargées à l'étape 101, trois paramètres représentatifs des performances du joueur. Ces trois paramètres peuvent être, par exemple, un paramètre P1 représentatif de la puissance développée par le joueur au cours de la dernière session de jeu, un paramètre P2 représentatif de l'endurance du joueur au cours de la période de jeu, et un paramètre P3 représentatif des aptitudes techniques du joueur, basé sur la régularité et la variété des coups effectués au cours de la période de jeu.

Dans une étape 103, sur la base des valeurs des paramètres P1, P2 et P3, le procédé affiche automatiquement, sur un écran 5 du dispositif d'affichage 3, les trois paramètres sur un diagramme comprenant trois axes formant une étoile à trois branches, chaque axe correspondant à l'un des paramètres P1, P2 et P3. Les valeurs respectives V1, V2 et V3 des paramètres P1, P2 et P3 sont représentées sur les axes P1, P2 et P3 par des points V1, V2 et V3.

Les trois points V1, V2 et V3 définissent un contour fermé A de forme globalement triangulaire qui englobe ou passe par les points V1, V2, et V3. Le terme « forme globalement triangulaire» signifie que le contour fermé A présente globalement trois parties allongées s'étendant respectivement entre les points V1 et V2, entre les points V1 et V3 et entre les points V2 et V3 et formant des angles entre elles.

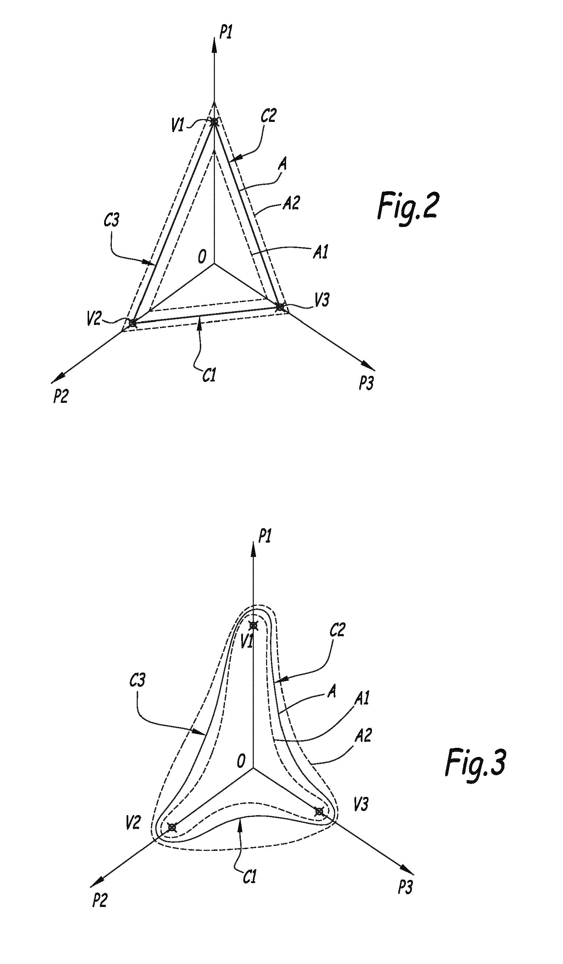

Dans un premier mode de réalisation représenté à la figure 2, le contour fermé A forme un triangle au sens géométrique du terme. Les points V1, V2 et V3 sont reliés par des côtés C1, C2 et C3 formés par des segments de droite. En variante non représentée, les côtés C1, C2 et C3 peuvent passer autour des points V1, V2 et V3.

Dans un second mode de réalisation représenté sur la figure 3, le contour fermé A comprend trois lignes courbes C1 et C2 et C3 s'étendant entre les points V1, V2 et V3, et reliées entre elles deux à deux autour des points V1, V2 et V3. En variante non représentée, les courbes C1, C2 et C3 peuvent également passer par les points V1, V2 et V3.

Dans une étape 104, qui peut être simultanée à l'étape 102, le temps de jeu effectif T du joueur de tennis sur la dernière session de jeu est calculé à partir des données D téléchargées à l'étape 101. Le temps de jeu effectif T est calculé, lors de l'acquisition des données par le système de mesure 2, en mesurant l'intervalle de temps séparant chaque coup exécuté par le joueur. Lorsque cet intervalle de temps est supérieur à une durée prédéterminée, indiquant que le joueur n'est plus effectivement en train de frapper des coups, cet intervalle de temps n'est pas considéré comme du temps de jeu effectif et n'est pas pris en compte.

En fonction de la mesure du temps de jeu effectif T effectuée à l'étape 104, le contour fermé A représenté à l'étape 103 est modifié dans une étape 105 afin d'augmenter et diminuer alternativement l'aire qu'il délimite selon une fréquence de variation F prédéterminée. La fréquence de variation est appelée F dans la suite « fréquence de battement ». L'aire du contour fermé A varie entre une aire minimale délimitée par un contour A1 et une aire maximale délimitée par un contour A2.

Dans le premier mode de réalisation représenté à la figure 2, les variations d'aire sont obtenues en augmentant et en diminuant la longueur des côtés C1, C2 et C3, en effectuant des homothéties du contour fermé A.

Dans le second mode de réalisation représenté à la figure 3, les variations d'aire du contour fermé A sont obtenues en modifiant la courbure des courbes C1, C2 et C3 et/ou en modifiant la longueur totale du contour fermé A, notamment au voisinage des points V1, V2 et V3.

Les diverses façons d'afficher le contour fermé A et de faire varier l'aire qu'il délimite peuvent être combinées dans le cadre de l'invention. Il est par exemple possible d'afficher le contour fermé A sous la forme d'un triangle dont les sommets sont les points V1, V2 et V3, et de modifier son aire en transformant ses côtés rectilignes C1, C2 et C3 en lignes courbes ayant des rayons de courbure variables.

La fréquence de battement F du contour fermé A est limitée à une valeur maximale F₀, par exemple 150 battements par minute, correspondant à 150 variations d'aire par minute.

La fréquence de battement maximale F₀ du contour fermé A est atteinte lorsque la dernière session de jeu dont les données D sont téléchargées comprend un temps de jeu effectif maximal supérieur à une valeur prédéterminée ΔT1, par exemple quarante minutes. Cela signifie que par exemple, si le joueur télécharge une session de jeu d'une heure, correspondant à quarante-cinq minutes de jeu effectif T, le temps de jeu effectif pris en compte est quarante minutes et déclenche un « battement » du contour fermé A à 150 battements par minute.

Le « battement » du contour fermé A a une durée limitée dans le temps. Si le joueur n'apporte pas de nouvelles données D pendant un certain temps ΔT2 après le dernier téléchargement de données, par exemple une semaine, la fréquence de battement F décroît jusqu'à une valeur prédéterminée, par exemple 110 battements par minute. Si après une nouvelle période de temps ΔT3, par exemple une semaine, le joueur n'a toujours pas téléchargé de nouvelles données D dans le système de calcul 4, la fréquence de battement F tombe à zéro.

Les délais à partir desquels la fréquence de battement F commence à décroître et le nombre de paliers utilisés peuvent être différents.

Si la fréquence de battement F du contour fermé A est égale à une valeur inférieure à la valeur maximale, le téléchargement d'une session de jeu comprenant un temps de jeu effectif T non nul mais inférieur à quarante minutes permet d'augmenter la fréquence de battement F pendant une certaine période de temps. Par exemple, un temps de jeu effectif T de dix minutes permet de prolonger la période pendant laquelle le contour fermé A va « battre » pendant une période de temps ΔT4 égale à quatre jours. Si au moment où ces dix minutes de jeu sont prises en compte par le système de calcul, la fréquence de battement F est égale à 50 battements par minute, la prise en compte de cette nouvelle quantité de temps de jeu effectif T permet également d'augmenter la fréquence de battement F à 100 battements par minutes, à titre d'exemple.

Dans une étape 106 qui a lieu après l'étape 101 et qui peut être simultanée aux étapes 102 à 105, chaque coup réalisé par le joueur lors de la période de jeu est classé dans un type de coup défini parmi une liste L de types de coups comportant au moins deux types de coups. Dans le présent exemple, la liste L de types de coups comprend quatre types de coups, au nombre desquels le coup droit, le revers, le service et le smash. En variante, la liste L de types de coups peut comprendre plus de quatre coups. On affecte à chaque type de coup la variable i, i étant compris entre 1 et 4.

De façon optionnelle, le procédé peut comprendre une étape 109 ayant lieu avant l'étape 106 et qui consiste à choisir les types de coup de la liste L de types de coups.

Dans une étape 107, sur une période de jeu donnée, le système de calcul 4 détermine le nombre de coups Ni exécutés pour chaque type de coup i en ajoutant, lors de chaque téléchargement de données à l'étape 101, les nombres de coups de chaque type de coup joués lors de chaque session de jeu dont les données sont téléchargées. Ainsi, le système de calcul 4 comporte pour chacun des quatre types de coups i de la liste L de coups, le nombre de coups total Ni effectué par le joueur depuis sa première utilisation de la raquette.

Dans une étape 108, à partir des nombres de coups Ni calculés à l'étape 106, un niveau de jeu du joueur est déterminé. Pour obtenir ce niveau, le joueur doit avoir effectué un nombre prédéterminé de coups dans chaque type de coup. Initialement, le joueur se situe au niveau zéro et évolue vers le niveau 1 une fois qu'il a effectué, par exemple, un nombre CD1 de coups droits, un nombre RE1 de revers, un nombre SE1 de services et un nombre SM1 de smashs. Par exemple, les nombres CD1, RE1, SE1 et SM1 sont respectivement égaux à 200, 200, 50 et 5. Pour passer au niveau 2, le joueur doit effectuer un nombre CD2, par exemple 500, de coups droits, un nombre RE2, par exemple 400 de revers, un nombre SE2, par exemple 100, de services et un nombre SM2, par exemple 20, de smashs. Le passage à chaque niveau supérieur est conditionné par l'exécution de nombre de coups prédéterminés pour chaque type de coup.

Pour représenter le niveau du joueur sur le diagramme du dispositif d'affichage 3, la surface délimitée par le contour fermé A est colorée par une teinte de couleur prédéterminée. Par exemple, le niveau 0 est représenté par la couleur bleu foncé.

A chaque fois que le joueur atteint, pour chaque type de coup i, le nombre de coups CD1, CD2, ..., RE1, RE2, ..., SE1, SE2, ..., SM1, SM2, ... requis pour accéder au niveau supérieur, le dépassement des nombres de types de coups déclenche le changement de teinte de couleur de la surface délimitée par le contour fermé A pour adopter la couleur correspondant au niveau supérieur. Le nombre prédéfini de coups pour chaque type de coup requis pour évoluer vers le niveau supérieur est augmenté à de nouvelles valeurs.

Si un joueur classé dans un certain niveau a effectué suffisamment de coups i dans un ou plusieurs des types de coups i, mais qu'il n'a pas exécuté suffisamment de coups dans au moins un autre type de coup, il ne peut passer au niveau supérieur.

A titre d'exemple, le système de calcul 4 peut être paramétré de manière que sept niveaux puissent être atteints. Dans ce cas, sept couleurs différentes sont prévues pour l'aire de la surface délimitée par le contour A. Ainsi, un rapide coup d'oeil à l'écran 5 permet au joueur de connaître son niveau.

Lorsque la fréquence de battement F du contour fermé A décroît jusqu'à atteindre zéro, les nombres de coups Ni pour chaque type de coups i accumulés par le joueur commencent également à décroître. Plus le temps T' écoulé depuis l'arrivée de la fréquence de battement F à zéro augmente sans que des données D soient téléchargées, plus le joueur perd un nombre de coups importants pour chaque type de coup. A titre d'exemple, au bout d'une semaine après l'arrivée de la fréquence de battement à zéro, le joueur peut perdre 25% des coups accumulés dans chaque type de coup. Ainsi, son niveau peut décroitre s'il ne joue pas pendant une période ΔT5 égale, dans l'exemple, à une semaine.

Selon un aspect optionnel, le système de calcul 4 peut prévenir le joueur un certain temps, par exemple un jour, avant que la fréquence de battement n'atteigne la valeur zéro, par exemple par un message qui s'affiche sur l'écran 5 du dispositif d'affichage 3.

Selon un autre aspect optionnel de l'invention, le dispositif d'affichage 3 peut être relié à un réseau tel qu'Internet, afin que différents joueurs puissent comparer leurs performances. De préférence, la moyenne des valeurs des trois paramètres P1, P2 et P3 est calculée, dans le dispositif d'affichage 3 ou bien dans un autre système, notamment avec un site internet, lors d'une étape 110. Ensuite, les valeurs moyennes calculées à l'étape 110 sont comparées entre elles lors d'une étape 111 afin de classer les joueurs les uns par rapport aux autres en fonction de leur performance. L'étape 111 est de préférence réalisée sur un site internet auquel les joueurs se connectent et sur lequel ils peuvent télécharger les données les concernant.

En variante, les comparaisons entre joueurs peuvent également être faites à partir des nombres de coups Ni dans chaque type de coup i, des niveaux, ou des fréquences de battement F du contour fermé A des différents joueurs.

Selon un mode de réalisation non représenté de l'invention, le procédé de traitement est apte à calculer plus de trois paramètres représentatifs des performances du joueur. En plus des paramètres P1, P2 et P3, le système de calcul 4 peut notamment calculer des paramètres représentatifs, par exemple du niveau de stratégie du joueur, de son niveau d'agressivité, ou de son niveau mental. Dans un tel cas, la forme du contour fermé A affiché à l'étape 103 est globalement polygonale et le diagramme comprend autant d'axes que de paramètres calculés à l'étape 102.

Les caractéristiques des différents modes de réalisation et variantes décrits ci-dessus peuvent être combinées pour générer de nouveaux modes de réalisation de l'invention.

## Revendications

1. Procédé de traitement de données (D) représentatives des performances d'un joueur de tennis par un dispositif d'affichage (3), **caractérisé en ce qu'**il comprend des étapes consistant à :
- a) calculer (102), à partir de données (D) comprenant des grandeurs physiques mesurées (100) au cours d'une période de jeu comprenant une ou plusieurs sessions de jeu et téléchargées (101) dans un système de calcul (4) du dispositif d'affichage (3), au moins trois paramètres (P1, P2, P3) représentatifs des performances du joueur lors de cette période de jeu,
- b) afficher (103), sous la forme d'un diagramme comprenant un axe pour chaque paramètre (P1, P2, P3), les axes formant une étoile à plusieurs branches, des points correspondant aux valeurs (V1, V2, V3) des paramètres sur les axes de manière à former un contour fermé (A) de forme globalement polygonale englobant ou passant par lesdits points,
- c) augmenter (A2) et diminuer (A1) alternativement (105) l'aire délimitée par le contour fermé (A) selon une fréquence de variation (F) calculée en fonction du temps de jeu effectif (T) du joueur mesuré (104) au cours de la dernière session de jeu de la période de jeu.

2. Procédé de traitement selon la revendication 1, **caractérisé en ce qu'**il comprend des étapes supplémentaires consistant à :
- d) déterminer (106), pour chaque coup réalisé lors de la période de jeu, le type de ce coup parmi une liste (L) de types de coups (i) prédéfinie comportant au moins deux types de coups,
- e) comptabiliser (107), pour chaque type de coup (i) de la liste (L) de coups, le nombre total (Ni) de coups réalisés ;
- f) colorer (108) une surface délimitée par le contour fermé (A) dans une couleur représentative du niveau du joueur, ce niveau étant déterminé en fonction du nombre total (Ni) de coups réalisés pour chaque type de coup (i) de la liste (L) de types de coups.

3. Procédé de traitement selon la revendication 2, **caractérisé en ce que** la liste (L) de types de coups (i) comprend au moins quatre types de coup, parmi lesquels le coup droit, le revers, le service et le smash.

4. Procédé de traitement selon l'une des revendications 2 et 3, **caractérisé en ce qu'**il comprend une étape consistant à signaler l'atteinte par le joueur d'un nombre (CD1, CD2, RE1, RE2, SE1, SE2, SM1, SM2) prédéfini de coups (Ni) pour chaque type de coup (i) par un changement de couleur de la surface délimitée par le contour fermé (A) et l'adoption d'une teinte correspondant à un niveau supérieur par rapport au niveau dans lequel le joueur était avant d'atteindre les nombres de coups prédéfinis pour chaque type de coup.

5. Procédé de traitement selon l'une des revendications précédentes, **caractérisé en ce que** les paramètres calculés à l'étape a) sont respectivement représentatifs de la puissance (P1) développée par le joueur au cours de la période de jeu, de l'endurance (P2) du joueur au cours de la période de jeu et des aptitudes techniques (P3) du joueur au cours de la période de jeu, et **en ce que** le contour fermé (A) a une forme globalement triangulaire.

6. Procédé de traitement selon l'une des revendications précédentes, **caractérisé en ce que** la fréquence de variation (F) de l'aire délimitée par le contour fermé (A) est limitée à une valeur maximale (F₀) qui est atteinte lorsque le temps de jeu effectif (T) lors de la dernière session de jeu de la période de jeu atteint une valeur seuil (ΔT1).

7. Procédé de traitement selon l'une des revendications précédentes, **caractérisé en ce que** la fréquence de variation (F) de l'aire délimitée par le contour fermé (A) décroît lorsque le joueur ne télécharge pas de données (D) pendant une période de temps (ΔT2) prédéterminée.

8. Procédé de traitement selon les revendications 2 et 7, **caractérisé en ce que** lorsque la fréquence de variation (F) devient nulle, les nombres totaux (Ni) de coups pour chaque type de coup (i) commencent à décroitre.

9. Procédé de traitement selon l'une des revendications précédentes, **caractérisé en ce que**, chaque fois que des données (D) concernant une nouvelle session de jeu sont téléchargées par le joueur dans le dispositif d'affichage (3), les variations de l'aire du contour fermé (A) sont prolongées pendant une période de temps (ΔT4) calculée en fonction du temps de jeu effectif (T) mesuré pendant cette dernière session de jeu.

10. Procédé de traitement selon l'une des revendications précédentes, **caractérisé en ce qu'**il comprend une étape (110) consistant à calculer une valeur moyenne des au moins trois paramètres (P1, P2, P3) calculés à l'étape a) et une étape (111) consistant à comparer, au moyen d'un réseau de données, les performances de différents joueurs de tennis sur la base de cette valeur moyenne.
